# EUROPEAN PATENT APPLICATION

(11) **EP 3 942 948 A1**
(43) Date of publication of application: **26.01.2022**
(21) Application number: 21174799.3
(22) Date of filing: 19.05.2021
(51) Int. Cl.: A24F 40/20, A24B 15/16, A61K 9/00

(54) **HEATED-SMOKABLE PRODUCT CONTAINING CANNABIS EXTRACT AND PREPARATION METHOD THEREOF**

(30) Priority: 16.04.2021 US 202117232205
(71) Applicant: Hmist Technology (Fujian) Co., Ltd., Xiamen, Fujian (CN)
(72) Inventor: Liu, Yiming, Xiamen (CN); Ye, Zihang, Xiamen (CN); Hao, Yunjia, Xiamen (CN)
(74) Representative: Loo, Chi Ching

(57) **Abstract**

A heated-smokable product contains a cannabis extract and a preparation method thereof. The heated-smokable product has a chamber for accommodating an atomization material, which is prepared from the following raw materials in parts by weight: 10-30 parts of cannabis extract, 25-33 parts of extracted-cannabis residues, 25-32 parts of tea medium and 18-36 parts of smoke producing agent. The preparation method reduces the content of miscellaneous smoke and potential hazards since flavors and fragrances are not added; the small-molecule cannabis extract and other components are jointly heated and atomized to significantly improve the natural fragrance and functionality of plants, and through atomization, fine and adequate smoke is produced with good smoking quality. The preparation method combines the extracted cannabis extract with the cannabis extraction residues, so that the cannabis extract as a pure natural substance is separated from and then returned to the original flowers and leaves.

## Description

### BACKGROUND OF THE INVENTION

### 1. Technical Field

The present invention belongs to the technical field of preparation and production of heated-smokable products, and in particular, relates to a heated-smokable product containing a cannabis extract and a preparation method thereof.

### 2. Description of Related Art

With the increasing improvement in the living standards of people, more and more consumers have paid attention to the smoking experience and sensory quality of heated-smokable tobacco products. To improve the smoking experience and sensory quality, flavors and fragrances are commonly added during the production of such products, which brings a certain adverse effect to the safety and functionality of such products.

In recent years, the research and development on combining natural extracts with atomization have started becoming a trend. Due to unique aromas and various medicinal effects, cannabis has a broad development prospect in the market segment of atomized products. However, there are few reports on the preparation of heated-smokable products from cannabis at present in the market, and it is of great significance to develop a heated-smokable product containing cannabis with good smoking experience and high smoking quality.

### BRIEF SUMMARY OF THE INVENTION

An object of the present invention is to provide a heated-smokable product containing a cannabis extract with good smoking experience and high smoking quality, and a preparation method thereof.

To achieve the object of the present invention, the present invention provides a heated-smokable product containing a cannabis extract, wherein the heated-smokable product has a chamber for accommodating an atomization material, which is prepared from the following raw materials in parts by weight: 10-30 parts of cannabis extract, 25-33 parts of extracted-cannabis residues, 25-32 parts of tea medium and 18-36 parts of smoke producing agent. The heated-smokable product is a product that does not burn under heat, i.e., heat not burning (HNB). When a special means is used for heating and a user inhaled to allow air to pass by, the product may produce aerosol that is derived from the evaporated atomization material contained in the product.

Preferably, the smoke producing agent is a mixture of glycerin and propylene glycol, the atomization material is prepared from the following raw materials in parts by weight: 12 parts of cannabis extract, 30 parts of extracted-cannabis residues, 30 parts of tea medium, 8 parts of glycerin and 20 parts of propylene glycol.

Preferably, the cannabis extract is full-spectrum cannabis oil containing no tetrahydrocannabinol (THC).

Preferably, the cannabis extract is tetrahydrocannabinol.

The present invention further provides a preparation method of the heated-smokable product containing a cannabis extract. The preparation method includes the following steps:
pulverizing dried cannabis flowers and leaves into flower-leaf particulate matters having a particle size of 300-350 meshes;
extracting the particulate matters to obtain a cannabis extract and extracted-cannabis residues, and drying the extracted-cannabis residues;
adding a smoke producing agent to the cannabis extract to obtain an extract diluent;
obtaining a tea medium;
stirring and mixing the tea medium, the extracted-cannabis residues and the extract diluent evenly to prepare a granular or flaky atomization material; and
filling the atomization material in a chamber of the smokable product.

Preferably, the dried cannabis flowers and leaves are prepared by drying fresh cannabis flowers and leaves at the drying temperature of 40-50°C for a period of 28-35 hours.

Preferably, the extracted-cannabis residues have a moisture content of 10% to 15% after being dried.

Preferably, a process of extracting the flower-leaf particulate matters to obtain the cannabis extract comprises a step of supercritical extraction, a step of screening through a molecular sieve and a step of rotary evaporation and condensation, in sequence.

Preferably, the tea medium is tea particles having a particle size of 300-350 meshes, and the tea particles are prepared by crushing off-the-shelf tea or by drying and crushing picked fresh tea.

Preferably, the granular atomization material has a particle size of 1-3 mm; and the amount of the atomization material filled to the chamber is 0.02 kg.

Compared with the prior art, the present invention at least has the following beneficial effects:
1. the heated-smokable product containing the cannabis extract according to the present invention is prepared from the raw materials including the cannabis extract, the extracted-cannabis residues, the tea medium and the smoke producing agent without the addition of flavors and fragrances, which reduces the content of miscellaneous smoke and potential hazards; the small-molecule cannabis extract and other components are jointly heated and atomized to significantly improve the natural fragrance and functionality of plants, and through atomization, fine and adequate smoke is produced with good smoking quality;
2. in the heated-smokable product containing the cannabis extract according to the present invention, the ratios among the cannabis extract, the extracted-cannabis residues, the tea medium and the smoke producing agent are carefully selected to allow liquid materials to be completely absorbed/adsorbed by solid materials without oil leakage or bleeding, and the tea medium can assist smoke production and can be mixed with the mellow aroma of the cannabis to product a special aroma, which greatly improves the smoking quality;
3. in the preparation method of the heated-smokable product containing the cannabis extract according to the present invention, the cannabis extract is first extracted and then combined with the extracted-cannabis residues, so that the cannabis extract as a pure natural substance is separated from and then return to the original flowers and leaves, resulting in outstanding manifestation of safety and functionality; and the preparation method has a simple process that is applicable to industrial homogeneous production in large scale and has good market and economic values;
4. in the preparation method of the heated-smokable product containing the cannabis extract according to the present invention, the cannabis flowers and leaves are pulverized into particles of 300-350 meshes, which ensures the extraction yield of the cannabis without any effect on the forming of the atomization materials, thereby reaching an efficient balance; and
5. the present invention applies the fine biochemistry technology to the tobacco industry, and obtains the cannabis extract by performing a step of supercritical extraction, a step of screening through a molecular sieve and a step of rotary evaporation and condensation in sequence, whereby good extraction effect and high yield are achieved, and the market prospect is broad.

### DETAILED DESCRIPTION OF THE INVENTION

An object of the present invention is to provide a heated-smokable product containing a cannabis extract with good smoking experience and high smoking quality, and a preparation method thereof.

For clearer illustration, the present invention is explained in detail below in conjunction with embodiments. It should be understood that the embodiments as described herein are only intended to explain, instead of limiting, the present invention. Unless otherwise specifically stated, the raw materials, reagents or devices used in the present invention are commercially available.

### Embodiment 1

This embodiment provides a heated-smokable product containing a cannabis extract and a preparation method thereof, wherein the cannabis extract is full-spectrum cannabis oil excluding tetrahydrocannabinol (THC).

Cannabis contains nearly 500 active ingredients, with at least 113 cannabinoids which can bind to some receptors in a human body to produce medical or other effects. Common ingredients of the cannabis extract include cannabidiol (CBD), tetrahydrocannabinol (THC), cannabidiolic acid (CBDA), cannabidivarine (CBDV), cannabinol (CBN), terpenes, volatile oil, flavonoids, etc.

These cannabis extracts have high bioactivity and diverse functions, and are not addictive except for etrahydrocannabinol (THC), so that the application prospect is broad. Among them, the cannabidiol (CBD) is particularly prominent due to its effects such as resistance to anxiety and depression, epilepsy, convulsions, oxidation, psychosis, nausea and vomiting, as well as tumor and cancer, protection of cranial nerves, and alleviation of pain and inflammation, is helpful to maintain the endocannabinoids inside a human body at a reasonable level to make a person feel good and happy, but is not as addictive as tetrahydrocannabinol (THC).

In this embodiment, the heated-smokable product prepared from the full-spectrum cannabis oil excluding the tetrahydrocannabinol (THC) can not only produce a good smoking effect, but also give effective play to the medical care and other effects of the full-spectrum cannabis oil excluding the tetrahydrocannabinol (THC).

The heated-smokable product containing a cannabis extract (i.e., the full-spectrum cannabis oil excluding tetrahydrocannabinol (THC)) has a chamber for accommodating an atomization material, which is prepared from the following raw materials in parts by weight: 12 parts of cannabis extract, 30 parts of extracted-cannabis residues, 30 parts of tea medium, 8 parts of glycerol and 20 parts of propylene glycol. All the components were carefully selected to allow liquid materials to be completely absorbed/adsorbed by solid materials without oil leakage or bleeding.

The preparation method of the heated-smokable product is as below.

S1, dried cannabis flowers and leaves were pulverized into flower-leaf particulate matters having a particle size of 300-350 meshes, more preferably 300 meshes.

In Step S1, fresh cannabis flowers and leaves were dried at a drying temperature of 40-50°C, preferably 45°C, for a period of 28-35 hours to obtain dried cannabis flowers and leaves. In this way, some acid substances existing in the fresh cannabis flowers and leaves could be removed, and active ingredients in the flowers and leaves could be prevented from being destroyed. Since the content of tetrahydrocannabinol (THC) was lowest in pistillate cannabis flowers, the pistillate cannabis flowers, more preferably pistillate flowers of industrial hemps (with the THC content of less than 0.3%), were preferred as the fresh cannabis flowers and leaves in this embodiment. The cannabis flowers and leaves might be pulverized by using an ultrafine pulverizer at a speed of 10,000-15,000 rpm for a period of 4-12 min. The pulverization meshes of 300-350 were comprehensively selected based on two aspects, namely, material forming and extraction yield. In one aspect, undersize particles from pulverization were not conductive to the later preparation of granular or flaky atomization material; and especially when the flowers and leaves were pulverized to 500 meshes or more, fibers were too fine to prepare the granular atomization material with the size of 2.00 mm, and a binder and the like were required to be added additionally for assisting forming, which, alternatively, might rely on a more complicated process or a special device. In the other aspect, oversize particles from pulverization are not conductive to the later extraction of cannabis extract. In this embodiment, the particle size range of 300-350 meshes is preferably selected through a plurality of experimental comparisons, with 300 meshes more preferred.

S2, the particulate matters were extracted to obtain a cannabis extract and extracted-cannabis residues, and the extracted-cannabis residues were dried.

In Step S2, the flower-leaf particulate matters were extracted. An extraction process might be water extraction, alcohol extraction and other processes that were commonly used in the art, and supercritical extraction was preferred in this embodiment. Specifically, a process of obtaining the cannabis extract included a step of supercritical extraction, a step of screening through a molecular sieve and a step of rotary evaporation and condensation in sequence, wherein for the supercritical extraction, a supercritical carbon dioxide extraction process was used with an extraction yield of 20%-25%. After extraction, a primary extracting solution and extracted-cannabis residues were obtained. In the step of screening through the molecular sieve, the molecular sieve was set to 314.4617 for the molecular weight of THC, thereby separating and removing THC in the primary extracting solution; and the resulting extracting solution was evaporated and condensed in a rotary evaporator at 90-95°C for 3-4 hours to obtain full-spectrum cannabis oil excluding the tetrahydrocannabinol (THC), i.e., the final cannabis extract in this embodiment. The extracted-cannabis residues are dried through dehydration drying or freeze-drying to reach a moisture content of 10%-15%, which is conductive to the later stirring and mixing and the absorption/adsorption of the cannabis extract and the smoke producing agent by the extracted-cannabis residues.

S3, a smoke producing agent was added to the dried cannabis extract to obtain an extract diluent.

In Step S3, the smoke producing agent was a mixture of glycerin and propylene glycol, with a weight ratio of the glycerin to the propylene glycol in the mixture as 2:5. The smoke producing agent might be either a commercially available product, or prepared based on the weight parts in this embodiment.

S4, a tea medium was obtained.

In Step S4, the tea medium was tea particles, which preferably had a particle size of 300-350 meshes, more preferably 300 meshes, for the combination with the extracted-cannabis residues. The tea particles were prepared by crushing dried tea. The dried tea might be commercially available off-the-shelf tea (leaves), for example, Tie Guanyin, Oolong tea, Maojian tea, Longjing tea, Pu'er tea or other common tea in the market, or might be a mixture of several common off-the-shelf tea, or might be prepared by drying picked fresh tea. It is easily conceivable that the sequence of executing this step was not unique, and might also be executed before Step S1 or concurrently with Step S1 or Step S2 or Step S3.

S5, the tea medium, the extracted-cannabis residues and the extract diluent were stirred and mixed evenly to prepare a granular or flaky atomization material.

In Step S5, the extract diluent from Step S3 was added to the tea medium from Step S4 and the extracted-cannabis residues from Step S2 and then stirred and mixed, and the added amounts of respective substances were as follows in parts by weight: 40 parts of the extract diluent (consisting of 12 parts of the full-spectrum cannabis oil excluding tetrahydrocannabinol (THC), 8 parts of glycerin and 20 parts of propylene glycol), 30 parts of the extracted-cannabis residues and 30 parts of the tea medium. The extract diluent, the tea medium and the extracted-cannabis residues after being stirred and mixed evenly were prepared into a granular or flaky atomization material. As a preference, the atomization material as particles has a particle size of approximately 2 mm, and as flakes, has a length of 12.55 mm and a thickness of 0.17 mm. It is easily conceivable that the particle size and the flake dimensions here were not uniquely defined, but can be adaptively adjusted according to customer needs and the size of the chamber of the smokable product.

S6, the atomization material was filled in the chamber of the smokable product.

In Step S6, the amount of filled atomization material was preferably 0.02 kg per product.

From the perspective of the absorption of the active ingredients, the heated-smokable product according to this embodiment has a gastrointestinal absorption efficiency of 25% and an alveolar absorption efficiency of 95% as compared with traditional cannabis products that are administered through gastrointestinal absorption. The active ingredients with higher purity and fewer impurities can be better improved in reaction efficiency. In this embodiment, the small molecules of the extract are combined with aerosol for smoking to achieve an effect that is far higher than a traditional medicinal effect, and moreover, the mellow aroma of the cannabis can be produced to create a better smoking experience. Different from other atomized products, the heated-smokable product according to this embodiment produces aerosol having softer and smoother smoking quality, and shows good effects such as resistance to anxiety and depression, epilepsy, convulsions, oxidation, psychosis, as well as nausea and vomiting, protection of cranial nerves, and alleviation of pain and inflammation.

In the preparation method of the heated-smokable product according to this embodiment, the cannabis extract as a pure natural substance is separated from and then return to the original flowers and leaves, resulting in outstanding manifestation of safety and functionality; and the preparation method has a simple process that is applicable to industrial homogeneous production in large scale and has good market and economic values.

### Embodiment 2:

This embodiment is the same as Embodiment 1 in terms of the preparation method of the heated-smokable product and the raw materials for preparing the atomization material, and differs from Embodiment 1 as follows: the atomization material is prepared from the following raw materials in parts by weight: 10 parts of cannabis extract, 25 parts of extracted-cannabis residues, 25 parts of tea medium and 18 parts of smoke producing agent.

### Embodiment 3:

This embodiment is the same as Embodiment 1 in terms of the preparation method of the heated-smokable product and the raw materials for preparing the atomization material, and differs from Embodiment 1 as follows: the atomization material is prepared from the following raw materials in parts by weight: 30 parts of cannabis extract, 33 parts of extracted-cannabis residues, 32 parts of tea medium and 36 parts of smoke producing agent.

### Embodiment 4:

This embodiment is the same as Embodiment 1 in terms of the preparation method of the heated-smokable product and the raw materials for preparing the atomization material, and differs from Embodiment 1 as follows: the atomization material is prepared from the following raw materials in parts by weight: 15 parts of cannabis extract, 28 parts of extracted-cannabis residues, 28 parts of tea medium and 20 parts of smoke producing agent.

### Embodiment 5:

This embodiment provides a heated-smokable product containing a cannabis extract and a preparation method thereof, wherein the cannabis extract is tetrahydrocannabinol (THC) oil.

Tetrahydrocannabinol (THC) naturally occurs in leaves and stems, pistillate flowers and seeds of Cannabis sativa L., with the highest content in seeds. The tetrahydrocannabinol (THC) may also be prepared by chemical synthesis. A cannabis product containing the tetrahydrocannabinol (THC) after being inhaled shows varying effects on the central nervous system depending on the dosage, the administration route and the special environment during medication, and has the manifestations of both excitement and inhibition, as well as disquieting thoughts after inhalation. The cannabis product has been used as an anesthetic in history, with an anesthetic component as the tetrahydrocannabinol (THC). The tetrahydrocannabinol (THC) has the effects of alleviating pain and inflammation, creating a feeling of relaxation and happiness, promoting sleep, causing excitement, assisting cancer chemotherapy recovery, acting as a diuretic, resisting to anxiety, depression, stress disorder, cancer, bacteria and the like, and thus has been applied to the medial industry.

In this embodiment, the heated-smokable product prepared by using tetrahydrocannabinol (THC) oil can not only produce a good smoking effect, but also provides medical benefits and other effects of the tetrahydrocannabinol (THC). From the perspective of the absorption of the active ingredients, the traditional medical application of the cannabis is based on the administration through gastrointestinal absorption, with a gastrointestinal absorption efficiency of 25% and an alveolar absorption efficiency of 95%. The active ingredients with higher purity and fewer impurities can be better improved in reaction efficiency. In this embodiment, the small molecules of the extract are combined with an aerosol for smoking to achieve an effect that is far higher than a traditional medicinal effect, and moreover, the mellow aroma of the cannabis can be produced to create a more pleasurable smoking experience. Different from other atomized products, the heated-smokable product according to this embodiment produces aerosol having softer and smoother smoking quality, and has effects of alleviating pain and inflammation, creating a feeling of relaxation and happiness, promoting sleep, causing excitement, assisting cancer chemotherapy recovery, and resisting to anxiety, depression, stress disorder.

In the preparation method of the heated-smokable product according to this embodiment, the cannabis extract as a pure natural substance is separated from and then return to the original flowers and leaves, resulting in outstanding manifestation of safety and functionality; and the preparation method has a simple process that is applicable to industrial homogeneous production in large scale and has good market and economic values.

This embodiment differs from Embodiment 1 as follows:
the cannabis extract is the full-spectrum cannabis oil containing no THC in Embodiment 1, and is the THC oil in this embodiment; and
in Step S2 of Embodiment 1, the THC oil separated by the molecular sieve is removed and discarded, and the full-spectrum cannabis oil excluding the THC oil is retained; and in this embodiment, the THC oil is retained in this embodiment, and the full-spectrum cannabis oil with the THC oil removed is discharged.

This embodiment is the same as Embodiment 1 in terms of other ingredients and ratios in the atomization material, and other steps in the preparation method of the heated-smokable product.

### Embodiment 6:

This embodiment is the same as Embodiment 5 in terms of the preparation method of the heated-smokable product and the raw materials for preparing the atomization material, and differs from Embodiment 5 as follows: the atomization material is prepared from the following raw materials in parts by weight: 10 parts of cannabis extract, 25 parts of extracted-cannabis residues, 25 parts of tea medium and 18 parts of smoke producing agent.

### Embodiment 7:

This embodiment is the same as Embodiment 5 in terms of the preparation method of the heated-smokable product and the raw materials for preparing the atomization material, and differs from Embodiment 5 as follows: the atomization material is prepared from the following raw materials in parts by weight: 30 parts of cannabis extract, 33 parts of extracted-cannabis residues, 32 parts of tea medium and 36 parts of smoke producing agent.

### Embodiment 8:

This embodiment is the same as Embodiment 5 in terms of the preparation method of the heated-smokable product and the raw materials for preparing the atomization material, and differs from Embodiment 5 as follows: the atomization material is prepared from the following raw materials in parts by weight: 15 parts of cannabis extract, 28 parts of extracted-cannabis residues, 28 parts of tea medium and 20 parts of smoke producing agent.

The embodiments as provided above are only to explain, instead of limiting, the technical solutions of the present invention. Although the above embodiments provide detailed description of the present invention, relevant technical staff should understand that the embodiments of the present invention can be modified or equivalently substituted. Without departing from the spirit and scope of the present invention, any modifications and equivalent substitutions should be construed as being covered by the scope of the Claims of the present invention.

## Claims

1. A heated-smokable product containing a cannabis extract, **characterized in that** the heated-smokable product has a chamber for accommodating an atomization material, which is prepared from the following raw materials in parts by weight: 10-30 parts of cannabis extract, 25-33 parts of extracted-cannabis residues, 25-32 parts of tea medium and 18-36 parts of smoke producing agent.

2. A heated-smokable product containing a cannabis extract as claimed in Claim 1, **characterized in that** the smoke producing agent is a mixture of glycerin and propylene glycol, the atomization material is prepared from the following raw materials in parts by weight: 12 parts of cannabis extract, 30 parts of extracted-cannabis residues, 30 parts of tea medium, 8 parts of glycerin and 20 parts of propylene glycol.

3. A heated-smokable product containing a cannabis extract as claimed in Claim 1 or Claim 2, **characterized in that** the cannabis extract is full-spectrum cannabis oil containing no tetrahydrocannabinol THC.

4. A heated-smokable product containing a cannabis extract as claimed in Claim 1 or Claim 2, **characterized in that** the cannabis extract is tetrahydrocannabinol THC.

5. A preparation method of a heated-smokable product containing a cannabis extract as claimed in any one of Claims 1 to 4, **characterized by** comprising the following steps:
pulverizing dried cannabis flowers and leaves into flower-leaf particulate matters having a particle size of 300-350 meshes;
extracting the particulate matters to obtain a cannabis extract and extracted-cannabis residues, and drying the extracted-cannabis residues;
adding a smoke producing agent to the cannabis extract to obtain an extract diluent;
obtaining a tea medium;
stirring and mixing the tea medium, the extracted-cannabis residues and the extract diluent evenly to prepare a granular or flaky atomization material; and
filling the atomization material in a chamber of the smokable product.

6. A preparation method of a heated-smokable product containing a cannabis extract as claimed in Claim 5, **characterized in that** the dried cannabis flowers and leaves are prepared by drying fresh cannabis flowers and leaves at the drying temperature of 40-50°C for a period of 28-35 hours.

7. A preparation method of a heated-smokable product containing a cannabis extract as claimed in Claim 5, **characterized in that** the extracted-cannabis residues have a moisture content of 10% to 15% after being dried.

8. A preparation method of a heated-smokable product containing a cannabis extract as claimed in Claim 5, **characterized in that** a process of extracting the flower-leaf particulate matters to obtain the cannabis extract comprises a step of supercritical extraction, a step of screening through a molecular sieve and a step of rotary evaporation and condensation, in sequence.

9. A preparation method of a heated-smokable product containing a cannabis extract as claimed in Claim 5, **characterized in that** the tea medium is tea particles having a particle size of 300-350 meshes, and the tea particles are prepared by crushing off-the-shelf tea or by drying and crushing picked fresh tea.

10. A preparation method of a heated-smokable product containing a cannabis extract as claimed in Claim 5, **characterized in that** the granular atomization material has a particle size of 1-3 mm; and the amount of the atomization material filled to the chamber is 0.02 kg.
